# EUROPEAN PATENT APPLICATION

(11) **EP 4 681 745 A1**
(43) Date of publication of application: **21.01.2026**
(21) Application number: 24189531.7
(22) Date of filing: 18.07.2024
(51) Int. Cl.: A61M 1/06

(54) **BREAST PUMP SYSTEM**

(71) Applicant: Koninklijke Philips N.V., 5656 AG Eindhoven (NL)
(72) Inventor: VAN DER KOOI, Johannes Tseard, 5656 AG Eindhoven (NL); LIPSCH, Job, 5656 AG Eindhoven (NL)
(74) Representative: Philips Intellectual Property & Standards

(57) **Abstract**

A breast pump system comprises a first kit and a second kit or a buffer. An actuator comprises back-to-back chambers on opposite sides of a divider (such as a piston), and a drive system is used for driving the actuator to change the first and second chamber volumes. The first kit is coupled to the first chamber and the second kit or buffer is coupled to the second chamber. One or both of the first kit and the second kit (if present) comprises a venting valve for venting to the atmosphere.

## Description

### FIELD OF THE INVENTION

This invention relates to breast pump systems, for example with an expression kit for each breast.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted. The breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

Often, it is desirable to express both breasts at the same time. A user will then typically acquire a set of identical devices (e.g. in one housing) to achieve a setup which performs stimulation and expression independently for each breast.

It has been recognized that when using two breast pumps, it is possible to improve the energy efficiency by transferring at least some of the vacuum pressure between the breast pumps instead of venting the vacuum pressure to the surroundings when the pressure is to be increased as part of a cyclic pressure waveform. For example, WO 2020/007671 and WO 2023/117684 each disclose a double breast pump device which enables vacuum to be transferred between two expression kits. Thus, the volume of one expression kit is used as a storage volume of under-pressure for the other expression kit. However, in each case a complicated valve arrangement is needed. The valves are relatively expensive and require a significant amount of energy. This partly cancels the energy saving benefits of re-using the vacuum. Furthermore, if a baseline vacuum is desired, more complex solutions are required.

### SUMMARY OF THE INVENTION

The invention is defined by the claims.

According to examples in accordance with an aspect of the invention, there is provided a breast pump system comprising:
a first kit;
a second kit or a buffer;
an actuator comprising a pair of back-to-back chambers, with a divider between them; and
a drive system for driving the actuator to change the volumes of the first and second chambers,
wherein the first kit is coupled to the first chamber and the second kit or buffer is coupled to the second chamber.

This system reduces cost by using an actuator to generate a pressure profile, by increasing and decreasing the volume of the first kit and the second kit or buffer. One volume is increased while the other is decreased. Thus, an increase in pressure in one volume is accompanied by a decrease in pressure in the other volume. This enables the vacuum to be shared and reused.

A first venting valve may be provided for venting the first kit to the atmosphere.

The venting valve for example opens when the pressure exceeds the atmospheric pressure. Thus, the venting valve prevents the pressure increasing above atmospheric pressure (but allows a pressure to be retained below atmospheric pressure).

The first venting valve may be part of the first kit, or it may be part of the actuator, or it may be in a coupling between the first kit and the actuator. The venting valve may vent the space between the breast and a kit membrane or the space outside the kit membrane. Furthermore, pressure release may be achieved by other means than a venting valve, such as by design of the interface between the expression kit and the breast.

The sum of the first and second chamber volumes is preferably constant. The relative sizes of the first and second chamber volumes determines the pressures on each side of the divider.

The arrangement avoids the need for a continuously operating pump to deliver an under-pressure, and it avoids the need for a complicated valve arrangement. In addition to simplifying the hardware, it reduces energy consumption by enabling the first kit and the second kit or buffer to be coupled together, so that instead of venting to the atmosphere in order to return from a vacuum level to a higher pressure level (e.g. atmospheric pressure), the pressure is at least partially reused. When two kits are used, they may be controlled with alternating cyclic pressure sequences.

The invention may be implemented as a system with two expression kits or with one kit and a buffer specifically provided for the purpose of reducing energy loss caused by venting pressure to the ambient surroundings.

The divider of the actuator is for example movable to change the first and second chamber volumes, and the sum of the first and second chamber volumes is constant. This means both chamber volumes can be controlled by a single actuator. The actuator functions as two back-to-back displacement pumps, controlled in anti-phase with each other.

The drive system for example comprises a controller which is configured to:
drive the divider from a first position to a second position, to increase the first chamber volume and decrease the second chamber volume, and thereby reduce the pressure at the first kit; and
return the divider to the first position.

This return may involve use of the pressure difference across the divider.

The energy required to drive the divider can be kept to a minimum by using the pressure difference across the divider to drive the divider back to (or towards) its neutral position.

The system may comprise a first kit and a second kit, wherein a second venting valve is provided for venting the second kit to the atmosphere, wherein the controller is configured to:
drive the divider from the first position to a third position, to decrease the first chamber volume and increase the second chamber volume, and thereby reduce the pressure at the second kit; and
return the divider to the first position.

Again, the return may involve use of the pressure difference across the divider.

Thus, a cyclic operation of the two kits is interleaved, with one experiencing an increase in pressure when the other experiences a decrease in pressure.

The controller is for example configured to set a speed and stroke length of the movement of the divider to create a desired vacuum profile.

The drive system for example comprises a linear motor.

The invention also provides a method of controlling a breast pump system for the expression of milk, wherein the breast pump system has a first kit, a second kit or a buffer, an actuator comprising a pair of back-to-back chambers, with a divider between them, and a drive system for driving the actuator to change the volumes of the first and second chambers, wherein the first kit is coupled to the first chamber and the second kit or buffer is coupled to the second chamber, wherein the method comprises:
controlling a drive system to drive the actuator from a first configuration to a second configuration, to increase the first chamber volume and decrease the second chamber volume, and thereby reduce the pressure at the first kit; and
controlling the drive system to return the actuator from the second configuration to the first configuration.

The milk expression is non-therapeutic.

This method uses control of volumes on opposite sides of a divider to generate a negative, i.e. below atmospheric, pressure, pressure waveform.

The divider of the actuator is for example movable to change the first and second chamber volumes, and wherein the sum of the first and second chamber volumes is constant, and the method comprises:
controlling a drive system to drive the divider from a first position to a second position, to increase the first chamber volume and decrease the second chamber volume, and thereby reduce the pressure at the first kit; and
controlling the drive system to return the divider from the second position to the first position.

The breast pump system for example comprises a first kit and a second kit, and the method further comprises:
controlling the drive system to drive the divider from the first position to a third position, to decrease the first chamber volume and increase the second chamber volume, and thereby reduce the pressure at the second kit while venting the first kit to the atmosphere if the pressure exceeds a threshold; and
controlling the drive system to release the divider such that it returns towards the first position under the pressure difference across the divider.

When controlling the drive system to drive the divider from the first position to the second position, the second kit may be vented to the atmosphere if the pressure exceeds a threshold.

The method may comprise setting a speed and stroke length of the movement of the divider to create a desired vacuum profile.

The invention also provides a computer program comprising computer program code means which is adapted, when said program is run on a controller of a breast pump system, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known breast pump system;
Fig. 2 shows a mother wearing two wearable breast pumps;
Fig. 3 shows a first breast pump system having a pump shared between two expression kits;
Fig. 4 shows operating cycles of the first breast pump system;
Fig. 5 shows operating cycles of a second breast pump system;
Fig. 6 shows a breast pump system with one expression kit and one buffer; and
Figs. 7 to 11 show operating phases for the system of Fig. 6.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system that comprises a first kit and a second kit or a buffer. An actuator comprises back-to-back chambers on opposite sides of a divider (such as a piston), and a drive system is used for driving the actuator to change the first and second chamber volumes. The first kit is coupled to the first chamber and the second kit or buffer is coupled to the second chamber. One or both of the first kit and the second kit (if present) comprises a venting valve for venting to the atmosphere.

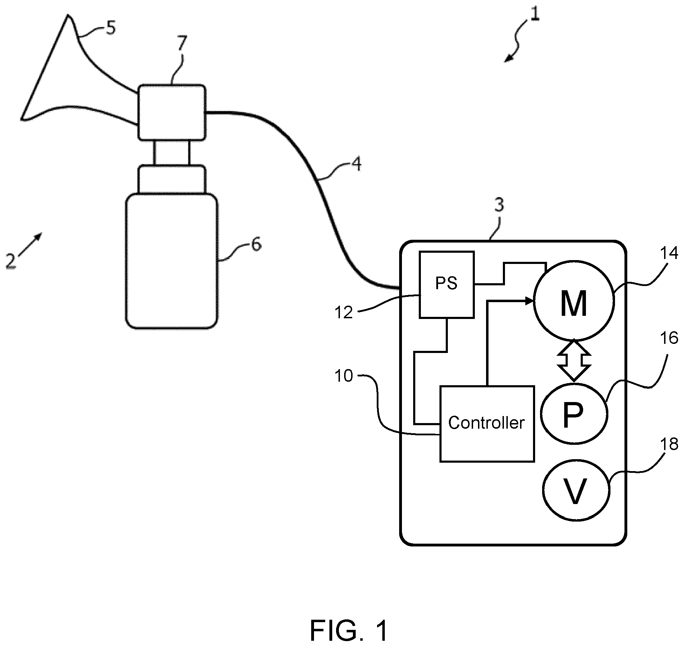

Fig. 1 shows a known breast pump system 1, comprising a single expression kit 2 and a pump arrangement 3 which are connected via a tube 4. The pump arrangement includes various components in addition to the pump, so may be considered to be a general operating unit.

The expression kit 2 is formed with a main body 7, a funnel 5 for receiving a breast of a user and a receptacle 6 for collecting the expressed milk. The funnel 5 and the receptacle 6 are connected to the main body 7. The main body 7 comprises a vacuum chamber. A flexible membrane or diaphragm is located in the vacuum chamber. The membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16. The vacuum pump comprises an impeller driven by the motor. The controller controls 10 the operation of the power source 12 and motor 14 (and hence the vacuum pump 16). The pump arrangement 3 further comprises a vacuum venting component, such as a solenoid valve 18.

In use, the vacuum pump applies a vacuum to the membrane located in the main body 7 so that it deforms. The membrane deforms to create a vacuum in the funnel 5 which in turns applies a vacuum to the breast which enables milk to be expressed. Although the breast pump system 1 is described as comprising a membrane such that the vacuum is applied indirectly to the breast, it should be understood that in an alternative embodiment, the vacuum is applied directly to the breast of a user. In this case, the breast pump system does not comprise a membrane and the vacuum created by the vacuum pump is applied directly to the breast.

The vacuum is applied to the breast at intervals. That is, a pressure differential is applied on a cyclic basis. In this known system, after a vacuum has been established, the pressure from the vacuum is vented by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression kit which enables milk to be expressed from the breast of a user. When the solenoid valve is opened, the vacuum generated by the vacuum pump is vented as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. In one type of design, a top part contains the drivetrain (pump, motor, controller) and a bottom part forms the collection vessel. This enables breast pumping to be performed more discretely. Of course, other designs are possible.

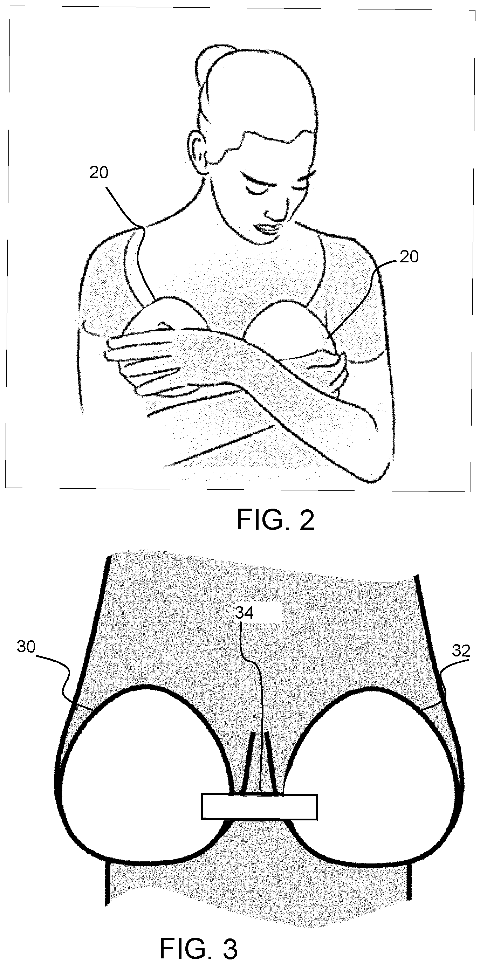

Fig. 2 shows a mother wearing two wearable breast pumps 20.

In such a design, the funnel 5 of Fig. 1 is implemented as breast shield, and this breast shield is received within an outer enclosure of the wearable breast pump.

The invention will be described with reference to a breast pump system having two expression kits. Each expression kit is an assembly for fitting over the breast of a user, and for example includes a breast shield and optionally also a membrane (known as the diaphragm) between the breast shield and the breast, so that the membrane limits the parts of the breast pump system that can be contaminated with milk.

The invention may however also be applied to breast pump systems with a single expression kit. The invention relates in particular to reducing energy loss when venting an expression kit to the ambient surroundings. When two expression kits are present, the invention involves equalizing pressure between them so that the pressure that would be vented can be considered to be re-used. However, a buffer (i.e. pressure storage vessel) may be used instead of the second expression kit to store energy as a vacuum rather than allowing it to be vented to the ambient pressure.

An example of the invention as applied to a breast pump system with two expression kits will first be described. It will then be explained how the invention may be applied to a system with one expression kit and a buffer.

Fig. 3 shows a breast pump system comprising a first expression kit 30 and a second expression kit 32. Each expression kit is a wearable device, and each expression kit includes a breast shield.

Instead of using a pump to generate an under-pressure, so that a vacuum profile can be created, the invention makes use of an actuator 34 coupled between the two expression kits. In known manner, the expression kits may each comprise a milk sensor for sensing milk flow and for measuring flow volume.

In a typical arrangement, the expression kits are independent and identical. However, it is also possible for expression kits to have different sets of components to reduce the duplication of hardware. For example, US 2020/155738 discloses a wearable breast pump with two expression kits, but only one has the controller or battery or pump to be used by both expression kits.

The overall system may be wearable (so that the actuator is located at one of the expression kits) or it may be portable, for example with the actuator and associated drive system remote from the expression kits, for example mounted on a belt.

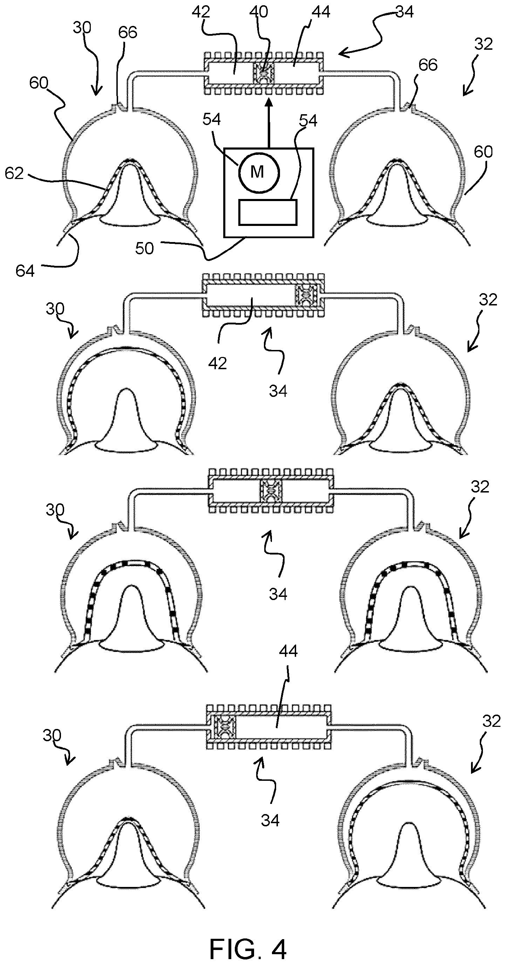

Fig. 4 shows the structure in more detail and also shows the operating phases of the breast breast pump system of Fig. 3.

The system comprises the first kit 30, the second kit 32 and the actuator 34.

The actuator comprises back to back chambers 42, 44, having first and second volumes respectively, on opposite sides of a divider. The total volume is constant, so that when one volume is increased, the other is decreased.

The simplest implementation of the actuator is in the form of a cylinder housing a piston (which is the divider), forming a pneumatic actuator. This is the example of actuator shown in Fig. 4. The movement of the piston changes the cylinder volumes on each side, so that a single drive signal controls both volumes. However, the actuator may be considered more generally to comprise two displacement pumps coupled together back-to-back. The term back-to-back is intended to denote the functionality rather than the geometrical layout, although the piston and cylinder layout is also geometrically back-to-back. The functional layout means that when one pump delivers an outlet pressure increase, the other delivers an outlet pressure reduction. The two pumps perform this in unison, and they work in anti-phase.

Instead of a piston and cylinder arrangement, bellows type pumps may be placed back-to-back. For example, a divider between them may move to simultaneously increase the volume on one side and decrease the volume on the other side. It is however also possible for the divider to be stationary, and the bellows on each side move in unison.

A drive system 50 is provided for driving the actuator, which in the example shown involves moving the piston 40 towards one end or the other end of the cylinder in which it is housed. The drive system comprises a motor 52 such as a linear motor and a controller 54. The motor for example couples to the piston using a magnet arrangement.

Each kit comprises an outer housing 60, and a membrane 62 between the outer housing 60 and the breast 64. Thus, each expression kit defines a first cavity over the breast of the user, and a second cavity which is separated from the first cavity by the membrane 62. The first cavity comprises a milk extraction space that connects to a milk collection chamber in a known manner.

The first kit 30 (in particular the space between the membrane and the outer housing) is coupled to the first chamber 42 and the second kit 32 (in particular the space between the membrane and the outer housing) is coupled to the second chamber 44.

In the example shown, each kit comprises a venting valve 66 for venting the space between the membrane and the outer housing to the atmosphere. This venting valve may instead be located at the (end of the) actuator or in the coupling between the kit and the actuator. The space between the membrane and the breast may instead be vented. Alternatively, an over-pressure release function may be implemented by the interface between the expression kit and the breast, so avoiding the need for a valve.

The over-pressure release function is for example used during a settling time before the system reaches a stable cyclic operation. The over-pressure release may also be needed because of the effective volume change of the expression kits resulting from the collection of milk.

The piston 40 controls the pressures in the two expression kits, in particular the pressures in the coupled space between the membrane and the outer housing. However, due to pressure equalization across the membrane, the same pressure is present against the breast.

The top image shows a neutral, central, position of the piston. Initially, the two expression kits are at atmospheric pressure because they have initially been placed over the breasts. The neutral position may however be non-central, and this may be used to enable a baseline under-pressure to be maintained within each expression kit at all times.

The operation of the breast pump is cyclic. There will be a start-up time, comprising a number of the cycles, before the pressures stabilize to create a repetitive sequence of pressure variations. For simplicity, the other cycles shown in Fig. 4 are based on the assumption that the breast pump has reached this stable operation.

The second image shows a position in which the piston has been driven by the drive system 50 from the first, neutral, position to a second position, to increase the first chamber volume 42 and decrease the second chamber volume 44, and thereby reduce the pressure at the first kit. The pressure at the second kit is increased. If this exceeds atmospheric pressure, it is vented to the atmosphere by the valve 66 of the second kit. Since the pressure preferably remains below atmospheric pressure in use (to prevent the breast shield falling off), the valves 66 are used during the start-up cycles.

The piston is then released such that it returns towards the first position under the pressure difference across the piston. This is shown as the third image. The pressures are equal in the two kits, at a pressure level between the atmospheric pressure (previously in the second kit) and the under-pressure (previously in the first kit).

The fourth image shows a position in which the piston has been driven by the drive system 50 from the first, neutral, position to a third position, to increase the second chamber volume and decrease the first chamber volume, and thereby reduce the pressure at the second kit. The pressure at the first kit is increased. If this exceeds atmospheric pressure, it is vented to the atmosphere by valve 66 of the first kit.

By repeating the four phases shown in Fig. 4, a cyclic pressure waveform is generated in each of the two expression kits. The speed and stroke length of the movement of the piston is used to create a desired vacuum profile.

If the pressures do not reach atmospheric pressure, the valves 66 will not need to open during normal operation (only during the initial start-up period), and a minimum pressure, below atmospheric pressure, may be maintained in the system at all times. One way to achieve this is to start (when the kits are both at atmospheric pressure because they have just been applied) with the piston non-centrally located, for example at one extreme end of the cylinder.

By not returning to that end position during the subsequent cycles, a baseline under-pressure can be maintained in the system. The same applies to both ends of the piston movement. If the extreme end position corresponds to atmospheric pressure in the kit on that side, if that end position is not reached in subsequent cycles, the pressure will remain below atmospheric pressure.

This non-central starting position effectively means the two kits may be considered to have different volumes (when the actuator chamber volumes are included), and this asymmetry enables a baseline under-pressure to be maintained. Another option is to have non-identical kits, in particular with different volumes.

The two expression kits may both be vented as shown. However, it is also possible to have only one expression kit vented, for example by starting in a non-central position as explained above. It can be ensured that one expression kit will not reach atmospheric pressure, even during the start-up cycles.

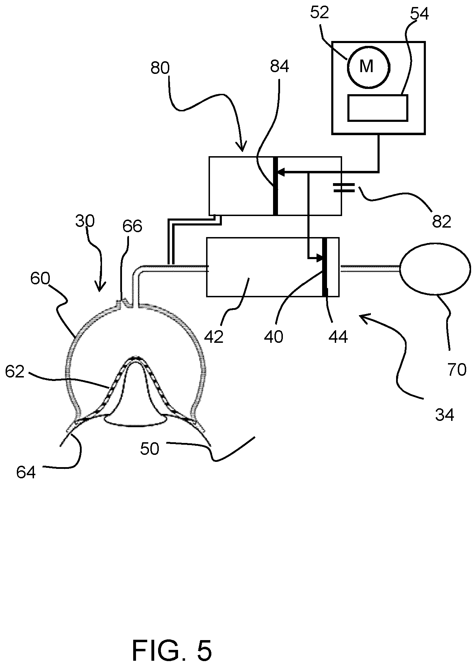

Fig. 5 shows an example with a first expression kit 30 and a buffer 70.

The buffer does not need a venting valve.

The system has a second actuator 80, again in this example it is shown as a cylinder and piston 84. The first and second actuators 34.80 are controlled independently. The second actuator is used so energy is not expended in creating an under-pressure in the buffer when increasing the pressure (i.e. venting) the expression kit.

The second actuator is coupled at one end to the expression kit 30 and at the other end to the atmosphere as shown at 82. Thus, first ends of the two actuators are coupled together and connected to the expression kit. One actuator connects at its other end to the buffer and the other is vented at its other end to the atmosphere.

The two actuators both comprise a divider which defines chambers on opposite sides. In the example shown, both actuators are pneumatic, with cylinders housing a piston (which is the divider).

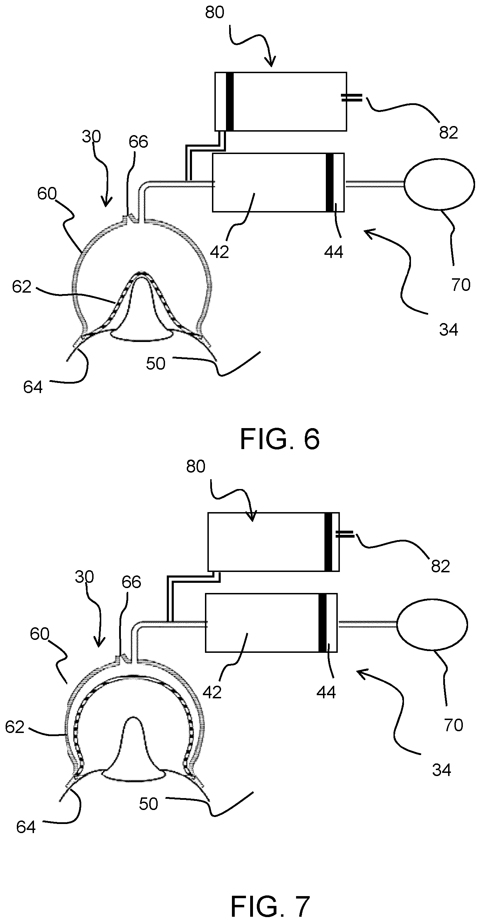

Figs. 6 to 11 show the operation cycles.

In Fig. 6, the expression kit 30 is applied. The first chamber volume 42 is maximal and the second chamber volume 44 is minimal. The second actuator 80 has its piston remote from the vent, so that the volume connected to the expression kit is minimal and the volume vented is maximal.

In Fig. 7, an under-pressure is applied to the expression kit 30 using the second actuator 80.

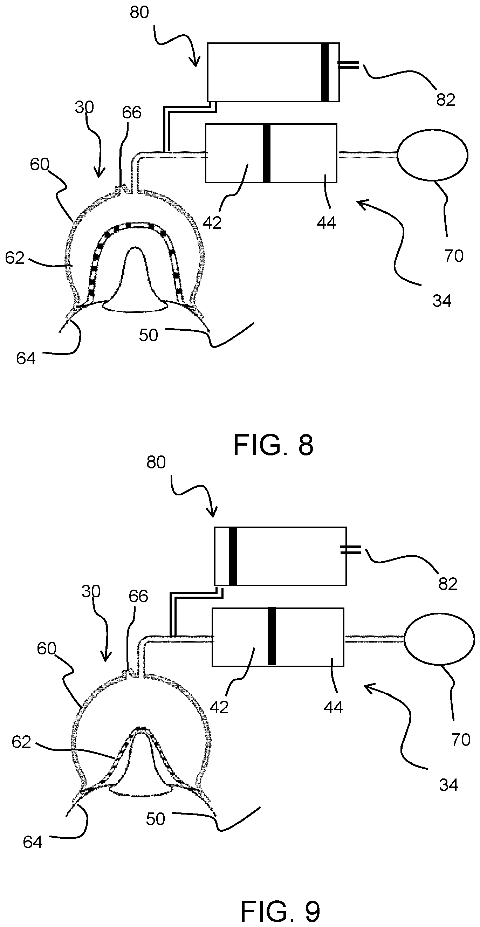

In Fig. 8, the under-pressure is partially released using the (first) actuator 34. This reuses pressure stored in the buffer 70.

In Fig. 9, the under-pressure is fully released by then using the second actuator 80. This re-uses pressure stored in the buffer 70.

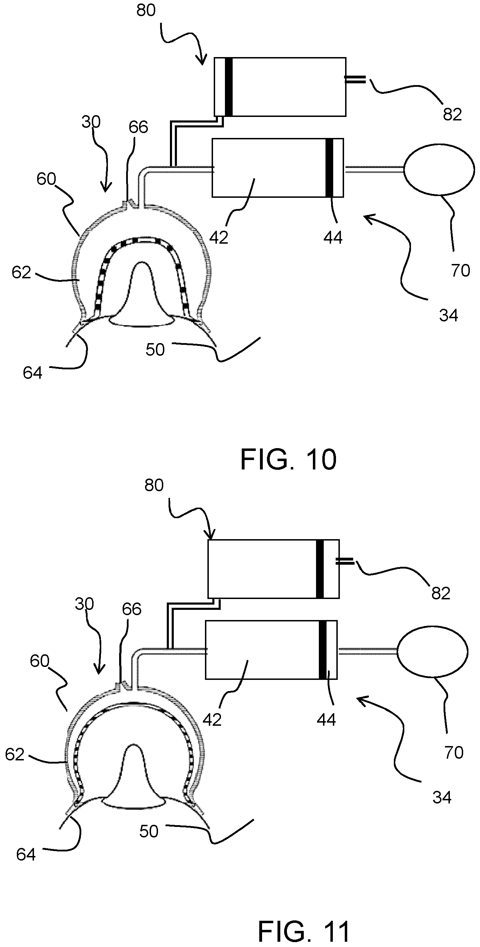

In Fig. 10, the under-pressure is re-applied (to an intermediate level) then using the (first) actuator 34.

In Fig. 11, the full under-pressure is achieved using the second actuator 80.

Fig. 11 corresponds to Fig. 7, so that Figs. 7 to 10 show the steps of a cyclic sequence.

It is noted that each step involves operation of one of the two actuators. During this time, the other actuator has its piston (i.e. divider) held stationary, so that the movement of one piston does not entrain movement of the other piston, thereby preventing the desired increase in vacuum or release of vacuum from the expression kit or buffer.

A cyclic pressure waveform may be applied to the first kit (and second kit if present) in the form of an expression waveform. However, the same approach may be applied to a stimulation waveform.

When there are cyclic expression and stimulation modes, they for example have different cyclic frequency and different maximum under-pressure (i.e. vacuum) levels.

The expression mode for example cycles between a baseline vacuum and a maximum vacuum (i.e., a maximum under-pressure) "Max_Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline_Vac = atmospheric pressure
Max_Vac = 300 mbar (30 kPa) below atmospheric pressure
TTV = 0.85 s
DI = 0.3 s
TTA = 0.05 s
DO = 0.35 s

This gives a total cycle time T_cycle of 1.55s.

These values are simply by way of example. The maximum under-pressure may be higher, for example 350 mbar (35 kPa) and the durations may differ. The cycle time is generally of the order of 1 to 2 seconds, typically 1 second (1 Hz).

The stimulation mode for example has a faster frequency hence with a shorter cycle time of around 0.5 seconds (2 Hz), and a lower vacuum, for example a maximum under-pressure level of 150 mbar (15 kPa).

These, or other, pressure profiles may be obtained by suitable control of the movement of the piston.

In the example above, part of the movement of the piston is achieved by releasing the piston and allowing the piston to move under the influence of the different pressures on each side. However, the piston may instead be positively driven to all of its different positions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump system comprising:
a first kit (30);
a second kit (32) or a buffer (70);
an actuator (34) comprising a pair of back-to-back chambers (40,42), with a divider (40) between them; and
a drive system (50) for driving the actuator to change the volumes of the first and second chambers,
wherein the first kit (30) is coupled to the first chamber (42) and the second kit (32), or buffer (70) is coupled to the second chamber (44).

2. The breast pump system of claim 1, wherein a first venting valve (66) is provided for venting the first kit (30) to the atmosphere.

3. The breast pump system of claim 1 or 2, wherein the divider of the actuator is movable to change the volumes of the first and second chambers, and wherein the sum of the first and second chamber volumes is constant.

4. The breast pump system of claim 3, wherein the drive system comprises a controller (54) which is configured to:
drive the divider (40) from a first position to a second position, to increase the first chamber volume and decrease the second chamber volume, and thereby reduce the pressure at the first kit; and
return the piston from the second position to the first position.

5. The breast pump system of claim 4, wherein the controller is configured to return the divider from the second position to the first position at least partly by releasing the divider (40) such that it returns towards the first position under the pressure difference across the piston.

6. The breast pump system of claim 4 or 5, comprising a first kit (30) and a second kit (32),
wherein a second venting valve (66) is provided for venting the second kit (32) to the atmosphere,
wherein the controller (54) is configured to:
drive the divider from the first position to a third position, to decrease the first chamber volume and increase the second chamber volume, and thereby reduce the pressure at the second kit; and
return the divider from the third position to the first position.

7. The breast pump system of claim 6, wherein the controller is configured to return the divider from the third position to the first position at least partly by releasing the divider (40) such that it returns towards the first position under the pressure difference across the piston.

8. The breast pump of any one of claims 4 to 7, wherein the controller (54) is configured to set a speed and stroke length of the movement of the divider to create a desired vacuum profile.

9. A method of controlling a breast pump system for expression of milk, wherein the breast pump system has a first kit (30), a second kit (32) or a buffer (70), an actuator (34) comprising a pair of back-to-back chambers, with a divider (40) between them, and a drive system (50) for driving the actuator to change the volumes of the first and second chambers, wherein the first kit is coupled to the first chamber and the second kit or buffer is coupled to the second chamber, wherein the method comprises:
controlling a drive system (50) to drive the actuator from a first configuration to a second configuration, to increase the first chamber volume and decrease the second chamber volume, and thereby reduce the pressure at the first kit; and
controlling the drive system to return the actuator from the second configuration to the first configuration.

10. The method of claim 9, wherein the divider of the actuator is movable to change the volumes of the first and second chambers, and wherein the sum of the first and second chamber volumes is constant, and the method comprises:
controlling a drive system (50) to drive the divider from a first position to a second position, to increase the first chamber volume and decrease the second chamber volume, and thereby reduce the pressure at the first kit; and
controlling the drive system to return the divider from the second position to the first position.

11. The method of claim 10, comprising controlling the drive system to release the divider thereby to return the divider from the second position towards the first position under the pressure difference across the piston.

12. The method of claim 10 or 11, wherein the breast pump system comprises a first kit and a second kit, wherein the method further comprises:
controlling the drive system to drive the divider from the first position to a third position, to decrease the first chamber volume and increase the second chamber volume, and thereby reduce the pressure at the second kit while venting the first kit to the atmosphere if the pressure exceeds a threshold; and
controlling the drive system to return the divider from the third position to the first position.

13. The method of claim 12, comprising controlling the drive system to release the divider thereby to return the divider from the third position towards the first position under the pressure difference across the divider.

14. A computer program comprising computer program code means which is adapted, when said program is run on a controller of a breast pump system, to implement the method of claims 9 to 13.

15. A processor which has been programmed using the computer program of claim 14.
